# EUROPÄISCHE PATENTANMELDUNG

(11) **EP 2 805 957 A1**
(43) Veröffentlichungstag der Anmeldung: **26.11.2014**
(21) Anmeldenummer: 13168851.7
(22) Anmeldetag: 23.05.2013
(51) Int. Cl.: C07F 3/00

(54) **Kristalline Formen von Trinatrium-Zink-Diethylen-triaminpentaessigsäure**

(71) Anmelder: hameln rds gmbh, 31789 Hameln (DE)
(72) Erfinder: Melnický, Radek, 785 01 ternberk (CZ); Lézar, Petr, 779 00 Olomouc (CZ); Hradil, Pavel, 783 14 Hlu ovice (CZ); Dewald, Mathias, 31787 Hameln (DE); Kakalik, Ivan, 900 81 enkvice (SK); Kralova, Janka, 900 01 Modra (SK); Smahovsky, Vendel, 902 01 Pezinok (SK); Valachovic, Pavol, 902 01 Pezinok (SK); Schickaneder, Christian, 91207 Lauf a.d.P. (DE)

(57) **Zusammenfassung**

Die vorliegende Erfindung betrifft die Herstellung von Trinatrium-Zink-Diethylentriaminpentaessigsäure (Zn-DTPA) und neue kristalline Formen dieses Salzes.

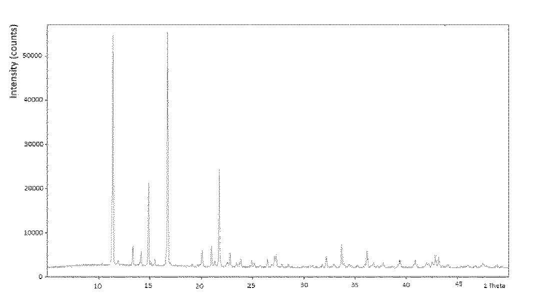

## Beschreibung

Die vorliegende Erfindung betrifft die Herstellung von Trinatrium-Zink-Diethylentriaminpentaessigsäure (Zn-DTPA) (Formel (I)) und kristalline Formen dieses Salzes.

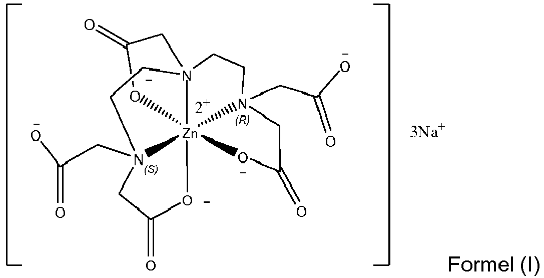

Die Erfindung betrifft ferner den Einsatz von Trinatrium-Zink-Diethylentriaminpentaessigsäure als Medikament und insbesondere eine Mensch- und/oder Tier oral verabreichbare Formulierung, umfassend die genannte Verbindung. Außerdem betrifft die Erfindung den Einsatz von Zn-DTPA oder eine diese Verbindung umfassende orale Formulierung zur Behandlung oder Prophylaxe von Intoxikation mit Schwermetallen und/oder Radionucliden.

### Stand der Technik

Diethylentriaminpentaessigsäure (DTPA) ist ein Chelatbildner. Chelatbildner werden in der Industrie standardmäßig verwendet, weil sie mit Metallen, teilweise selektiv, stabile Komplexe eingehen.

In der pharmazeutischen Industrie wird Diethylentriaminpentaessigsäure als Kalzium-oder Zink-Komplex verwendet. Diese Komplexe sind in der Lage, das Kation gegen ein anderes Kation auszutauschen, wenn dieses Kation eine größere Komplexbildungskonstante aufweist, d.h. einen stabileren Komplex mit dem Komplexbildner bildet. Lösungen dieser Salze werden in Europa und in den USA zur Komplexierung von Radionukliden verwendet. (Ménétrier F, Grappin L, Raynaud P, Courtay C, Wood R, Joussineau S, List V, Stradling GN, Taylor DM, Bérard P, Morcillo MA & Rencova J (2005) Treatment of accidental intakes of plutonium and americium: Guidance notes. Appl Radiat Isot, 62: 829-846.)

Fertige Arzneimittel mit Zn-DTPA sind unter dem Handelsnamen Pentetate Zinc Trisodium (USA, NDA August 2004, hameln pharmaceuticals) und Zink-Trinatrium-Pentetat (DE, Heyl Chemisch-pharmazeutische Fabrik GmbH & Co.) zugelassen.

Die Fertigarzneimittel werden durch intravenöse Injektion verabreicht. Sie sind indiziert für einen atomaren Zwischenfall, bei dem radioaktive Schwermetalle wie Plutonium, Americum und Curium frei gesetzt werden. Guilmette, R. A. et al "Effectiveness of continously infused DTPA therapy in reducing the radiation dose from inhaled 244Cm203 aerosols" Health Physics, 1992. vol. 62, no. 4, pp. 311-318.

Derzeit werden auch Anstrengungen unternommen, die orale Verfügbarkeit des Komplexes zu erhöhen (WO 2007/145682).

Für die intravenösen Darreichungsformen ist die Bildung des Zink-Komplexes in Lösung ein zufriedenstellender Syntheseweg. Die Verwendung für die perorale Anwendung stellt jedoch höhere Ansprüche an die physikalischen Eigenschaften des Wirkstoffs. Der Komplex muss in fester Form vorliegen. Er sollte bevorzugt eine stabile, leicht handhabbare Beschaffenheit aufweisen.

Die Synthese des Wirkstoffs wurde erstmals in DE1223396 (Beispiel 1) veröffentlicht. Die Standard-Methode zur Herstellung von Zn-DTPA besteht in der Bereitstellung einer wässrigen Lösung von Pentanatrium-Diethylentriaminpentaessigsäure und der Zugabe von Zink Chlorid oder Zinkoxid nach folgendem Schema:

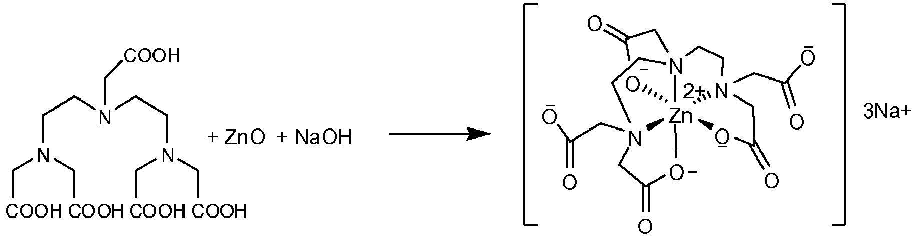

Dabei wird die Isolierung des Wirkstoffs über Konzentration der Reaktionslösung durch Abdestillieren des Wassers und anschließende Trocknung des Reaktionsproduktes erreicht.

Der in DE1223396 dargestellte Syntheseweg führt jedoch zu einem amorphen Produkt, dass sehr hygroskopisch und entsprechend schlecht zu vermahlen ist.

In einer Anmeldung von Nanotherapeutics wird die Hygroskopie ebenfalls als nachteilige Eigenschaft von Zn-DTPA dargestellt (WO2007008480, Beispiel 4). Es wird berichtet, dass das lyophilisierte Pulver innerhalb von 24 h mehr als 10 Gew.-% Wasser aufnimmt.

DE1223396 vermittelt keinerlei Hinweis auf die Existenz einer kristallinen Form, oder unterschiedlicher polymorpher Formen von Zn-DTPA.

**Tabelle 1** zeigt die Hygroskopie des Feststoffs, wenn das Produkt auf dem aus dem Stand der Technik bekannten Weg durch Aufkonzentration aus dem Reaktionsgemisch erhalten wird (vgl. auch Beispiel 1).

**Tabelle 1**

| | **Durchschnittlicher Massenzuwachs [%]** | | | | | | |
|---|---|---|---|---|---|---|---|
| **Zeit [h] No.** | **1** | **2** | **4** | **6** | **8** | **24** | **48** |
| Charge 1 | 0.51 | 0.89 | 1.56 | 2.35 | 2.95 | 7.38 | 11.92 |
| Charge 2 | 0.56 | 0.97 | 1.73 | 2.61 | 3.26 | 7.82 | 12.02 |

Nach 12 Stunden liegt der Massenzuwachs aufgrund von Hygroskopie bei etwa 7-8 %, nach 48 Stunden liegt der Massenzuwachs der Probe bei etwa 12 %. Dies ist etwa die Größenordnung, die im Stand der Technik für Zn-DTPA bekannt ist. Dabei ist die Hygroskopie von herkömmlichen Zn-DTPA so stark, dass es bei ausreichend langer Exposition gegenüber Luftfeuchtigkeit sogar aufgrund seiner Hygroskopie zerfließt.

Ca-DTPA bildet aufgrund der vergleichbaren Elektronenkonfiguration von Kalzium (Ca:[Ar]4s2; Zn:[Ar] 3d10 4s2) einen ähnlichen Komplex. Der Komplex ist jedoch aufgrund der unterschiedlichen Atomradien deutlich instabiler. Ein Vergleich der beiden Komplexe in ihren chemischen und physikalischen Eigenschaften ist daher schwierig.

Für die Herstellung von Ca-DTPA, wird eine Methode beschrieben, bei der zu einer kalten wässrigen Lösung des Produktes ein Alkohol zugegeben wird, um eine Ausfällung zu bewirken (GB944020). Eine kristalline Form dieses Komplexes ist aber auch hier nicht beschrieben.

Bisher ist es nicht gelungen, Zn-DTPA in einer Form darzustellen, die zu einem stabilen Produkt führt, dass unter atmosphärischen Bedingungen zu einer oralen, pharmazeutischen Darreichungsform verarbeitet werden kann.

Vor diesem Hintergrund war es Aufgabe der vorliegenden Erfindung, Zn-DTPA in einer Form bereitzustellen, die unter atmosphärischen Bedingungen stabiler als die bislang bekannte und darstellbare Form ist. Insbesondere war es Aufgabe der Erfindung, die Hygroskopizität von Zn-DTPA zu verlangsamen und/oder herabzusetzen. Außerdem sollte das erfindungsgemäße DTPA bevorzugt geeignet sein für orale pharmazeutische Darreichungsformen.

Diese Aufgabe wird gelöst durch Trinatrium-Zink-Diethylentriaminpentaessigsäure (Zn-DTPA) in kristalliner Form.

Sofern Zweifel an der Definition des Wortes "kristallin" bestehen sollten, so ist im Rahmen dieses Textes unter "kristallin" zu verstehen, dass in der entsprechenden Substanz Ionen oder Moleküle nicht zufällig, sondern regelmäßig in einer Kristallstruktur angeordnet sind. Dies zeigt sich im Röntgen-Diffraktogramm durch ein deutlich erkennbares Beugungsmuster. Beispiele für ein solches Beugungsmuster sind in den Figuren angegeben. Die darin gezeigten Reflexionen, wie z.B. in den Tabellen 1 und 2 dargestellt, sind für jede Kristallform reproduzierbar im gleichen 2 Theta-Winkel angeordnet. Wie bereits oben beschrieben waren bislang kristalline Formen von Zn-DTPA nicht zugänglich. Überraschenderweise hat sich herausgestellt, dass sich solche kristallinen Formen durch hier weiter unten beschriebene Verfahren herstellen lassen. Insbesondere hat sich überraschenderweise herausgestellt, dass bei den kristallinen Formen die Hygroskopie verlangsamt und insgesamt verringert ist. Auch hierzu wird auf die weiter unten gemachten Ausführungen verwiesen.

Bevorzugt liegt erfindungsgemäß Trinatrium-Zink-Diethylentriaminpentaessigsäure in einer kristallinen Form vor, wobei deren Pulver-Röntgen-Diffraktogramm einem Pulver-Röntgen-Diffraktogramm zuzuordnen ist, dass mindestens die folgenden charakteristischen Peaks aufweist (Form I):

**Tabelle 2**

| Pos. [°2Th.] | d-spacing [A] | Rel. Int. [%] |
|---|---|---|
| 6.8836 | 14,90 | 48 |
| 8.1807 | 12,54 | 100 |
| 13.781 | 7,46 | 7 |
| 14.477 | 7,10 | 52 |
| 17.725 | 5,81 | 43 |
| 20.567 | 5,01 | 4 |

oder das mindestens die folgenden charakteristischen Peaks aufweist (Form II):

**Tabelle 3**

| Pos. [°2Th.] | d-spacing [Å] | Rel. Int. [%] |
|---|---|---|
| | | |
| 11.302 | 9,08 | 21 |
| 11.384 | 9,02 | 86 |
| 14.892 | 6,90 | 32 |
| 16.699 | 6,16 | 100 |
| 21.721 | 4,75 | 41 |
| 33.734 | 3,08 | 10 |

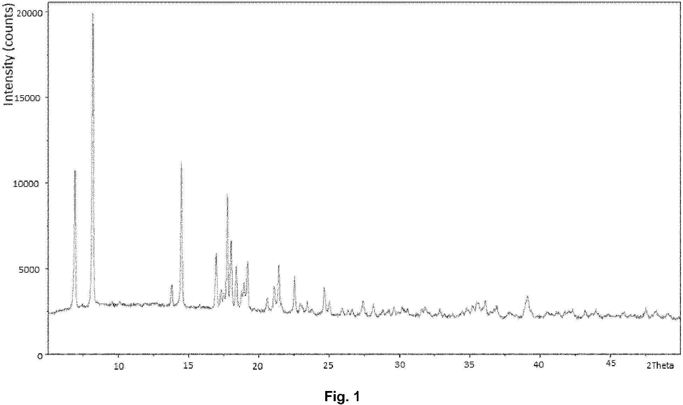

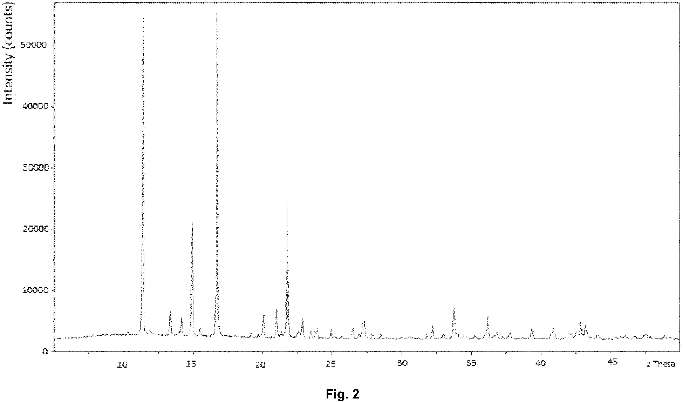

Die Röntgen-Diffraktogramme in Figur 1 und Figur 2 zeigen die beiden kristallinen Formen I und II. Sofern nicht anders angegeben, beziehen sich die Röntgen-Diffraktogrammangaben in diesem Text auf Aufnahmen, die mit Co K-alpha Strahlung gemacht wurden. Eine genaue Beschreibung der Röntgen-Diffraktogramm-Aufnahmen findet sich im Beispiel 7.

Dem Fachmann ist dabei klar, dass es - abhängig von den ganz konkreten Messbedingungen - zu leichten Abweichungen sowohl was die Peak-Lage als auch die relative Intensität der Peaks betrifft, kommen kann. Dementsprechend bedeutet im Sinne dieses Textes, dass ein "Pulver-Röntgen-Diffraktogramm einem Pulver-Röntgen-Diffraktogramm mit einer bestimmten Peak-Lage und Peakintensität zuzuordnen ist", dass der Fachmann in der Lage ist, festzustellen, dass die gemessene Probe dem bestimmten Peak-Muster und den entsprechenden Peak-Intensitäten unter Berücksichtigung der besonderen Messsituation entspricht. Dies bedeutet, dass die Beugungsmuster im Wesentlichen mit den Figuren der bevorzugten Kristallform übereinstimmen, bevorzugt die Peaks des Pulver-Röntgen-Diffraktogramms der bevorzugten erfindungsgemäßen Kristallformen jeweils um maximal ± 0,5 besonders bevorzugt ± 0,3 hinsichtlich ihrer Position, bevorzugt hinsichtlich ihres d-Wertes gegenüber den in diesem Text gemachten Angaben verschoben sind.

Form I entsteht z.B. wenn Methanol verwendet wird, um Zn-DTPA aus einer heißen, wässrigen Lösung auszufällen. Vergleiche auch Beispiel 2.

Form II entsteht beispielsweise, wenn das Wasser durch azeotrope Destillation mit n-Butanol aus dem Reaktionsgemisch entfernt wird (vergleiche z.B. Beispiel 5, unten), und das Produkt aus dem Reaktionsgemisch auskristallisiert. Eine umfassendere Peakliste für Form II ist weiter unten (Tabelle 5 angegeben, wobei es bevorzugt ist, dass über die oben angegebenen Peaks hinaus wenigstens einer, zwei, drei oder sogar alle in der nachfolgenden Tabelle zusätzlich angegebenen Peaks im Pulver-Röntgen-Diffraktogramm der erfindungsgemäßen kristallinen Form Zn-DTPA gefunden werden.

**Tabelle 4 Peakliste (Form II)**

| Pos. [°2Th.] | d-spacing [Å] | Rel. Int. [%] |
|---|---|---|
| | | |
| 11.302 | 9,08 | 21 |
| 11.384 | 9,02 | 86 |
| 14.892 | 6,90 | 32 |
| 16.699 | 6,16 | 100 |
| 20.038 | 5,14 | 6 |
| 20.962 | 4,92 | 8 |
| 21.721 | 4,75 | 41 |
| 22.840 | 4,52 | 6 |
| 26.436 | 3,91 | 3 |
| 27.139 | 3,81 | 4 |
| 27.291 | 3,79 | 5 |
| 28.494 | 3,63 | 1 |
| 32.168 | 3,23 | 5 |
| 33.734 | 3,08 | 10 |
| 34.426 | 3,02 | 1 |
| 36.161 | 2,88 | 8 |
| 36.777 | 2,84 | 2 |
| 42.811 | 2,45 | 6 |
| 43.183 | 2,43 | 5 |

Die erfindungsgemäßen kristallinen Formen von Zn-DTPA (insbesondere die bevorzugten) zeichnen sich außerdem durch hohe chemische Stabilität und eine geringe Hygroskopizität aus. Dabei ist das erfindungsgemäße Zn-DTPA nicht nur hinsichtlich seiner absoluten Hygroskopizität gegenüber den aus dem Stand der Technik bekannten Produkten überlegen, sondern auch hinsichtlich der Wasseraufnahmegeschwindigkeit.

Dementsprechend ist eine erfindungsgemäße Trinatrium-Zink-Diethylentriaminpentaessigsäure bevorzugt, wobei die Trinatrium-Zink-Diethylentriaminpentaessigsäure ausgehend von einem Wassergehalt von 8 Gew.-% bei Lagerung bei 65 % Luftfeuchtigkeit und 25 °C für 48 h ≤ 10 Gew.-% Wasser aufnimmt.

Bevorzugt ist dabei eine erfindungsgemäße Trinatrium-Zink-Diethylentriaminpentaessigsäure, die ausgehend von einem Wassergehalt von 8 Gew.-% bei Lagerung bei 65 % Luftfeuchtigkeit und 25 °C für 48 h ≤ 7 Gew.-% Wasser aufnimmt.

Eine verlangsamte Wasseraufnahme hat insbesondere den Vorteil, dass bei der Verarbeitung der erfindungsgemäßen kristallinen Form von Zn-DTPA weniger rigide Maßnahmen hinsichtlich einer Verringerung von Luftfeuchtigkeit benötigt werden, im Idealfall lassen sich die erfindungsgemäßen Formen sogar ohne besondere Maßnahmen z. B. zu pharmazeutischen Formulierungen weiterverarbeiten.

Die nachfolgende Tabelle 1 zeigt dabei den Vergleich zwischen konventionell hergestelltem amorphen Zn-DTPA und zwei erfindungsgemäßen kristallinen Formen. Dabei wurden die jeweils eingesetzten Proben dem gleichen Vortrocknungsprozess unterworfen (vgl. Beispiele 3 und 4). Die Proben wurden für den in der Tabelle jeweils angegebenen Zeitraum bei 25 °C und einer Luftfeuchtigkeit von 65 % relativer Luftfeuchte ausgesetzt.

Tabelle 5 zeigt den Vergleich zwischen konventionell hergestelltem amorphem Zn-DTPA und den beiden kristallinen Formen.

**Tabelle 5**

| **Zeit [h] No.** | **Durchschnittlicher Massenzuwachs [%]** | | | | | | |
|---|---|---|---|---|---|---|---|
| | **1** | **2** | **4** | **6** | **8** | **24** | **48** |
| Form I (aus Beispiel 2) | 0.32 | 0.54 | 0.92 | 1.34 | 1.64 | 3.91 | 6.81 |
| Form II, A (aus Beispiel 3) | 0.34 | 0.57 | 1.00 | 1.49 | 1.84 | 4.42 | 5.07 |
| Form II, B (aus Beispiel 4) | 0.41 | 0.72 | 1.29 | 1.97 | 2.43 | 5.18 | 5.25 |
| Amorph I (aus Beispiel 1) | 0.51 | 0.89 | 1.56 | 2.35 | 2.95 | 7.38 | 11.92 |
| Amorph II (aus Beispiel 1) | 0.56 | 0.97 | 1.73 | 2.61 | 3.26 | 7.82 | 12.02 |

Die erfindungsgemäßen kristallinen Formen zeigen also einen Massenzuwachs von weniger als 10 %, bevorzugt weniger als 7 %, besonders bevorzugt weniger als 6 % nach 48 Stunden unter den genannten Bedingungen.

Auch die absolute Wasseraufnahme der erfindungsgemäßen kristallinen Formen ist gegenüber den aus dem Stand der Technik bekannten amorphen Formen deutlich verbessert. Letztere neigen sogar dazu, bei ausreichend langer Exposition gegenüber Luftfeuchtigkeit zu zerfließen. Dementsprechend ist erfindungsgemäß bevorzugt eine erfindungsgemäße Trinatium-Zink-Diethylentetraaminpentaessigsäure, wobei diese nach Lagerung bei 65 % Luftfeuchtigkeit und 25 °C für 200 h im festem Zustand verbleibt.

Weiter bevorzugt ist eine erfindungsgemäße Trinatrium-Zink-Diethylentriaminpentaessigsäure, die nach Lagerung bei 65 % Luftfeuchtigkeit und 25 °C für 200 h einen Gesamtgehalt von ≤ 16 Gew.-% bevorzugt ≤ 15 Gew.-%, weiter bevorzugt ≤ 14 Gew.-% Wasser umfasst.

Der Vorteil der geringeren Hygroskopizität (absolut) liegt insbesondere darin, dass die erfindungsgemäßen kristallinen Formen zuverlässig als Festkörper verbleiben, während die aus dem Stand der Technik bekannten amorphen Formen von Zn-DTPA sogar zum Zerfließen neigen. Dadurch eröffnen sich durch die erfindungsgemäßen Formen eine Reihe weiterer Anwendungen.

Dementsprechend ist Teil der Erfindung eine erfindungsgemäße Trinatrium-Zink-Diethylentetrapentaessigsäure zur Verwendung als Arzneimittel. Wie bereits oben beschrieben waren bislang die kristallinen Formen nicht zugänglich und konnten dementsprechend - obwohl ein Bedarf bestand - auch nicht im medizinischen Bereich eingesetzt werden.

Vor diesem Hintergrund und insbesondere angesichts der verbesserten Eigenschaften (ganz besonders in Bezug auf die Hygroskopizität) ist Teil der Erfindung eine Mensch und/oder Tier oral verabreichbare, insbesondere pharmazeutische Formulierung, umfassend erfindungsgemäßes Zn-DTPA.

Eine Vielzahl von sinnvollen Formulierungen, insbesondere für die orale und ganz besonders als pharmazeutische Verabreichung sind aufgrund der verbesserten Stabilität des erfindungsgemäßen Zn-DTPA sinnvoll möglich. Eine oral verabreichbare Formulierung ist dabei eine Formulierung, die aufgrund ihrer Bestandteile und bevorzugt auch aufgrund ihrer Konsistenz einem Tier oder einem Menschen, insbesondere einem Menschen verabreicht werden kann. Eine pharmazeutische Formulierung ist in dem Zusammenhang eine Formulierung, die bevorzugt die an pharmazeutische Produkte gestellten Voraussetzungen insbesondere in Deutschland erfüllt.

Bevorzugt umfasst eine oral verabreichbare, insbesondere eine oral verabreichbare pharmazeutische Formulierung einen geeigneten Träger. Die Formulierung kann als Direktverpressung des Wirkstoffs verwirklicht werden oder weitere Hilfsmittel umfassen, ausgewählt aus der Gruppe bestehend aus Füllmitteln, insbesondere Stärke (Mais-Kartoffel- oder Weizenstärke), Lactose, Glucose, Mannitol oder Sorbitol; Bindemitteln, insbesondere MCC (mikrokristalline Cellulose) oder Stärke; Feuchtbindemitteln/Klebstoffen für die Granulierung, insbesondere Stärkekleister, Celluloseether, Kollidon oder Gelatine; Sprengmitteln, insbesondere Kartoffelstärke, Maisstärke, PVP, Carbopol oder Magnesiumperoxid und Gleitmitteln.

Teil der Erfindung ist auch der Einsatz von erfindungsgemäßem Zn-DTPA oder einer oral verabreichbaren Formulierung, umfassend erfindungsgemäßes Zn-DTPA zur Behandlung oder Prophylaxe von Intoxikation mit Schwermetallen und/oder Radionucliden. Dabei sind insbesondere Radionuclide die bevorzugte Anwendungsform. Besonders wirksam ist dabei das erfindungsgemäße Zn-DTPA bzw. die dieses umfassende Formulierung gegenüber den Schwermetallen und/oder Radionucliden ausgewählt aus der Gruppe bestehend aus Uran, Curium, Plutonium und Americum, wobei die beiden letztgenannten besonders bevorzugt sind.

Teil der Erfindung ist ein Verfahren zur Herstellung von erfindungsgemäßer Trinatrium-Zink-Diethylentriaminpentaessigsäure umfassend die Schritte:
a) Reaktion von Tetraethylpentaessigsäure mit Zinkoxid oder einem anderen Zinksalz in Gegenwart von Wasser,
b) zweimaliges Einengen der Reaktionslösung auf ≤ 15 % des Ausgangsvolumens und nachfolgende Zugabe eines organischen Lösungsmittels und
c) Isolieren des kristallinen Reaktionsproduktes.

Mit diesem erfindungsgemäßen Verfahren lässt sich insbesondere die oben beschriebene Form I hervorragend darstellen.

Teil der Erfindung ist auch ein Verfahren zur Herstellung von erfindungsgemäßer Trinatrium-Zink-Diethylentriaminpentaessigsäure, umfasst die Schritte
a) Reaktion von Tetraethylpentaessigsäure mit Zinkoxid oder einem anderen Zinksalz in Gegenwart von Wasser,
b) Entfernen von 90 - 99 Gew.-%, bevorzugt 94 bis 96 Gew.-% des Wassers aus dem Reaktionsgemisch, wobei 15 - 100 Gew.-%, bevorzugt 20 - 60 Gew.-%, besonders bevorzugt 20 bis 45 Gew.-%, organisches Lösungsmittel bezogen auf den Wassergehalt vor Beginn der Wasserentfernung parallel zur oder vor Beginn der Wasserentfernung zugesetzt wird,
c) ggf. Hinzufügen von weiterem organischem Lösungsmittel nach Schritt b),
d) weiteres Entfernen von Wasser und bevorzugt auch von organischen Lösungsmittel bis die Dampftemperatur im Bereich von ± 5°C der Siedetemperatur des organischen Lösungsmittels liegt und/oder bis Kristallisation eintritt und
e) Isolieren des kristallinen Reaktionsproduktes.

Dieses erfindungsgemäße Verfahren ist besonders geeignet für die Darstellung der kristallinen Form II.

Generell kann das erfindungsgemäße Zn-DTPA durch Neutralisation von Diethylentriaminpentaessigsäure in wässriger Lösung mittels Natriumhydroxid und nachfolgender Zinkoxidzugabe hergestellt werden. Auch andere Zinksalze als Zinkoxid sind möglich.

Für das erfindungsgemäße Verfahren ist es dabei vorteilhaft, wenn das (organische) Lösungsmittel so gewählt wird, dass die Löslichkeit von Zn-DTPA in dem Lösungsmittel geringer ist als 1 g/ml.

Weil das erfindungsgemäße Produkt in Wasser sehr gut lösbar ist, muss während der Herstellung des erfindungsgemäßen Produkts das Wasser aus der Reaktionsmischung entfernt werden (siehe oben).

Grundsätzlich ist es möglich, Wasser z. B. durch Vakuumdestillation zu entfernen, wobei vorher oder währenddessen ein geeignetes Lösungsmittel hinzugefügt werden sollte, um einen Teil des Wassers zu ersetzen. Wie oben beschrieben ist dabei bevorzugt, dass als solches Lösungsmittel 15 - 100 Gew.-%, bevorzugt 20 - 45 Gew.-% an organischem Lösungsmittel bezogen auf den Wassergehalt vor Beginn der Wasserentfernung zugegeben werden.

Besonders zuverlässig erhält man dabei die erfindungsgemäßen Produkte, wenn kontrolliert wird, wie viel Wasservolumen aus der Reaktionslösung entfernt wurde. Dies ist beispielsweise möglich durch Auffangen des Wassers, als Alternative ist aber auch die Destillationstemperatur ein guter Hinweis für den verbleibenden Wassergehalt in der Reaktionsmischung.

In diesem Zusammenhang ist azeotrope Destillation ein besonders geeignetes Wasserentfernungsverfahren, bei dem das Wasser schrittweise entfernt wird und der verbleibende Wasseranteil in der Reaktionsmischung umgekehrt proportional zur Temperatur ist.

Die azeotrope Destillation ist eine gängige Methode, um Wasser aus einem öligen Reaktionsprodukt zu entfernen, siehe auch EP1413575, Beispiel 5. Als Mittel der Wahl wird Toluol verwendet. Ein weiteres Beispiel für die Entfernung von Wasser aus einem Reaktionsprodukt durch azeotrope Destillation wird in EP0449445 beschrieben.

Dementsprechend ist es vorteilhaft, wenn das verwendete (organische) Lösungsmittel mit Wasser ein Azeotrop bildet. So ist die Entfernung des Wassers über die azeotrope Destillation ermöglicht, wobei bevorzugt so lange Wasser aus dem Reaktionsgemisch entfernt wird, bis das Produkt in dem Gemisch in der Hitze zu kristallisieren beginnen kann. Es ist dabei auch möglich, nach Erreichen des bevorzugten Wassergehaltes von nur noch etwa 1 - 10 Gew.-%, bevorzugt 4 - 6 Gew.-% des ursprünglich enthaltenen Wassers bzw. besonders bevorzugt von etwa 5 Gew.-% des ursprünglich enthaltenen Wassers noch einmal organisches Lösungsmittel zuzugeben.

Ferner kann es erfindungsgemäß bevorzugt sein, während der azeotropen Destillation das organische Lösungsmittel in die Reaktionslösung wieder zurückzuführen. Außerdem kann es in diesem Zusammenhang bevorzugt sein, nach Entfernen des Wassers im Rahmen der azeotropen Destillation auch organisches Lösungsmittel abzudestillieren.

Im Regelfall erfolgt die Destillation von Wasser und ggf. organischem Lösungsmittel so lange, bis sich eine honigartige Flüssigkeit ergibt, die sich bevorzugt an der Reaktoroberfläche niederschlägt und die bei zunehmender Wasserentfernung an Dichte gewinnt.

Bei bevorzugten Ausführungen des erfindungsgemäßen Verfahrens beginnt das Produkt sich bei 97 - 98 °C Dampftemperatur zu verfestigen.

Bevorzugt ist bei Erreichen einer Dampftemperatur von 110 °C (insbesondere im Rahmen einer azeotropen Destillation mit n-Butanol als organischem Lösungsmittel) das erfindungsgemäße kristalline Produkt ausgefallen.

Bevorzugt wird dieses nun durch einen Filtrationsschritt isoliert.

Die für die erfindungsgemäßen Verfahren einzusetzenden organischen Lösungsmittel sind bevorzugt ausgewählt aus der Gruppe bestehend aus Aceton, n-Butanol, 2-Methyl-1-propanol, Methanol, Ethanol, n-Propanol, 2-Propanol, 2-Methyl-2-propanol, Toluol und Ethylacetat.

Dabei ist besonders bevorzugt für die Herstellung der Kristallform I, dass Methanol das organische Lösungsmittel ist, während für die kristalline Form II besonders bevorzugt Toluol und am meisten bevorzugt n-Butanol das einzusetzende organische Lösungsmittel ist.

Es spricht aber auch nichts dagegen, dass andere Lösungsmittel, die mit Wasser ein Azeotrop bilden, für die Herstellung nach dem erfindungsgemäßen Verfahren verwendet werden. Wie bereits oben angedeutet ist die Entfernung des Wassers im erfindungsgemäßen Verfahren durch azeotrope Destillation vorteilhaft, sie ist jedoch nicht für jedes Lösungsmittel notwendig. Ähnliche Ergebnisse können auch erzielt werden, wenn die Reaktionslösung durch (beliebige) Destillation genügend eingeengt wird und anschließend ein (organisches) Lösungsmittel zur wässrigen Reaktionslösung hinzugegeben wird.

Bevorzugte Schritte für die erfindungsgemäßen Verfahren sind das Neutralisieren der eingesetzten Tetraethylpentaessigsäure mittels einer Natriumhydroxidlösung und/oder das Abkühlen des Reaktionsgemisches, insbesondere vor einer Filtration.

Da es aber grundsätzlich möglich ist, das Filtrat auch aus der heißen Suspension zu gewinnen, ist die Isolierung des erfindungsgemäßen Reaktionsproduktes verhältnismäßig schnell möglich, wobei Unreinheiten in der abfiltrierten Flüssigkeit verbleiben.

Somit ist es insbesondere im Rahmen des bevorzugten erfindungsgemäßen Verfahrens möglich, zwei Effekte auszunutzen: Zum einen handelt es sich dabei um die geringe Löslichkeit von Zn-DTPA in bestimmten organischen Lösungsmitteln und zum anderen die Fähigkeit bestimmter Lösungsmittel, ein Azeotrop mit Wasser zu bilden, um so die Wasserentfernungsbedingungen und den Wasserentfernungsfortschritt optimal zu kontrollieren. Diese Eigenschaften wurden zwar schon früher dafür eingesetzt, Wasser aus Reaktionsgemischen zu entfernen, es ist aber überraschend, dass es mittels der erfindungsgemäßen und insbesondere der bevorzugten erfindungsgemäßen Methoden möglich ist, Zn-DTPA in definierter, kristalliner Form mit reproduzierbaren Produkteigenschaften und einer verringerten Hygroskopizität und somit einer verbesserten Stabilität zu erhalten.

Das bevorzugte erfindungsgemäße Verfahren in Form der azeotropen Destillation besitzt insbesondere folgende Vorteile:
- Es ist verhältnismäßig leicht, die Menge des verbleibenden Wassers in der Reaktionsmischung zu bestimmen (aufgrund der Temperatur-Proportionalität), insbesondere wenn man mit anderen Wasserentfernungsmethoden wie z. B. Vakuumdestillation vergleicht. Das gleiche gilt natürlich auch für die Menge an aus der Reaktionsmischung entferntem Wasser.

- Bei Einsatz der bevorzugten Lösungsmittel, insbesondere bei n-Butanol in Zusammenhang mit einer azeotropen Destillation liegt ein Vorteil in der geringen Löslichkeit des Produktes in dem Lösungsmittel und darüber hinaus in einer hohen Reaktionsausbeute.
- Ferner ist es möglich, das organische Lösungsmittel, insbesondere n-Butanol, zurückzugewinnen, was die Herstellungskosten reduziert.
- Besonders vorteilhaft an dem erfindungsgemäßen Verfahren ist, dass nur eine polymorphe Form erhalten wird.

### Beispiele

### Beispiel 1: Herstellung von amorphem Zn-DTPA

152,5 g NaOH wurden in 1,7 L Wasser gelöst und die Lösung auf 30 °C abgekühlt. Anschließend wurden 500 g DTPA unter Rühren zugegeben und gerührt, bis eine klare Lösung entstand (etwa 20 min). 103,4 g Zinkoxid wurden zur Lösung gegeben und die Lösung über Nacht gerührt, bis eine klare Lösung entstand.

Der pH-Wert wurde mit einer 10 %igen NaOH-Lösung auf 7.0 eingestellt und die Lösung filtriert. Anschließend wurde die Lösung im Vakuum bei 40 °C unter starkem Rühren eingeengt und bei 90 °C bei 2,5 KPa für 17 Stunden getrocknet.

Ergibt 641,1 g, Entspricht 96,5 % der Theorie. Reinheit nach HPLC: 99,6 %; Wassergehalt: 7,16 %.

### Beispiel 2: Herstellung von kristallinem Zn-DTPA Form I

296 g NaOH wurden in 3,4 L Wasser gelöst und die Lösung auf 30 °C abgekühlt. Anschließend wurden 1 kg DTPA unter Rühren zugegeben und gerührt, bis eine klare Lösung entstand (etwa 20 min). 207 g Zinkoxid wurden zur Lösung gegeben und die Lösung über Nacht gerührt, bis eine klare Lösung entstand.

Der pH-Wert wurde mit einer 10 %igen NaOH-Lösung auf 7.01 eingestellt und die Lösung filtriert. Anschließend wurde die Lösung im Vakuum bei 40 °C unter Rühren eingeengt und 3,2 L Methanol zugegeben. Die Lösung wurde erneut eingeengt, so dass 3 Liter Flüssigkeit abdestilliert wurden. Es wurden erneut 2 Liter Methanol zugegeben, so dass ein Feststoff auskristallisierte. Die Kristalle wurden abfiltriert und bei 90 °C bei 2,5 KPa für 17 Stunden getrocknet.

Ergibt 812,4g, d.h. 61,14% der Theorie. Reinheit nach HPLC: 99,6 %; Wasser nach Karl Fischer: 5,49 %.

### Beispiel 3: Herstellung von kristallinem Zn-DTPA Form II

In einer Apparatur mit Wasserabscheider wurden 296 g NaOH in 3,4 L Wasser gelöst und 1 kg DTPA unter Rühren zugegeben. 207 g Zinkoxid wurde zugegeben, und der pH-Wert auf 7,0 eingestellt. 1 Liter n-Butanol wurden zu der Suspension gegeben und die Lösung so lange unter Abscheidung von Wasser (azeotrope Destillation) erhitzt, bis eine Kristallisation eintrat. Nach Abkühlung der Reaktionslösung wurde das kristalline Produkt abfiltriert.

Trocknung des Produktes bei 90 °C bei 2,5 KPa für 17 Stunden ergibt 1,3 kg, entspricht 100 % der Theorie; Gehalt nach HPLC 101,6 %, Wassergehalt: 8 %.

### Beispiel 4: Herstellung von kristallinem Zn-DTPA Form II

In einer Apparatur mit Wasserabscheider wurden 296 g NaOH in 3,4 L Wasser gelöst und 1 kg DTPA unter Rühren zugegeben. 207 g Zinkoxid wurde zugegeben, und der pH-Wert auf 7,0 eingestellt. Etwa 2,5 Liter Wasser wurden abdestilliert. Dann wurde 1 Liter n-Butanol zu der Lösung gegeben und so lange unter Abscheidung von Wasser erhitzt, bis eine Kristallisation eintrat. Nach Abkühlung der Reaktionslösung wurde das kristalline Produkt abfiltriert.

Trocknung des Produktes bei 90 °C bei 2,5 KPa für 17 Stunden ergibt 1,2 kg, entspricht 92 % der Theorie; Gehalt nach HPLC 100,0 %, Wassergehalt: 7,6 %.

### Beispiel 5:

5 kg Diethylentriaminopentaessigsäure wurden in eine Lösung von 1,48 kg Natriumhydroxid in Wasser (17 kg) gegeben. Danach wurde Zinkoxid 1,04 kg hinzugefügt und die Reaktionsmischung gerührt.

Als nächster Schritt wurde Diatomit (1,0 kg) hinzugefügt und die Suspension durch einen geeigneten Filter gefiltert und nachfolgend mit Wasser gewaschen. Nun wurden 22 kg Butanol dem Filtrat hinzugefügt und diese Mischung einer azeotropen Destillation unter atmosphärischem Druck unterworfen.

Das Wasser wurde während der Destillation abgetrennt und das Volumen des abgetrennten Wassers sowie die Destillationstemperatur regelmäßig geprüft. Die n-Butanol-Phase wurde zurück ins Reaktionsgefäß geführt und während der azeotropen Destillation schrittweise 6 kg weiteres N-Butanol in die Reaktionsmischung hinzugefügt, um das entfernte Wasser teilweise zu ersetzen.

Bei Erreichen einer Temperatur von 101 - 102 °C wurde weiteres n-Butanol (12 kg) hinzugefügt und die Destillation fortgesetzt, bis eine Temperatur von 110 °C im Reaktionsgemisch erreicht wurde. Nun wurde auch kein n-Butanol mehr in die Reaktionsmischung geführt und alles n-Butanol abgetrennt.

Die kochende Suspension wurde gerührt und das Reaktionsprodukt mittels Filtration separiert. Hierbei ist es grundsätzlich möglich, die Reaktionsmischung abzukühlen oder sie heiß zu filtrieren.

Schließlich wurde das Endprodukt in einem Vakuumtrockner bei 90 °C getrocknet und das erwünschte Produkt (Kristallform II) erhalten.

Die verbleibende Flüssigkeit sowie die Butanol-Phase konnten destilliert werden und das zurückerhaltene Butanol für die nächste Charge verwendet werden.

### Beispiel 6: Bestimmung der Hygroskopizität

Die Hygroskopizität von Zn-DTPA wurde geprüft durch Messung der Gewichtszunahme in einer kontrollierten Umgebung. Bei (25 ± 2) °C Lufttemperatur und (60 ± 5) % Luftfeuchtigkeit in einer Stabilitätskammer (Thermo TEC) wurden die Gewichte von jeweils zwei Proben (5 .000g) kontrolliert (Analytische Waage, XS 205, Mettler Toledo). Das Gewicht wurde nach 1, 2, 4, 6, 8, 24 und 48 Stunden notiert.

### Beispiel 7: Ermittlung der Pulver-Röntgen-Diffraktogramme

Die Pulver-Röntgen-Diffraktogramm-Muster der festen Formen Zn-DTPA wurden innerhalb des 2Theta-Bereichs 2° bis 51° auf einem Bragg-Brentanofokusierpulver-Diffraktometer, Philips, Modell 1730/10 (Philips, Holland) ermittelt, wobei das Diffraktometer an einen PC zur weiteren Auswertung angeschlossen war.

Das Instrument war mit einer Röntgenstrahlröhre ausgerüstet, die Co Kα Strahlung, Wellenlänge 0,179021 nm bereitstellt. Die Messbedingungen waren wie folgt: Anregungsspannung: 40 kV, Anodenstromstärke: 35 mA, Schrittgröße: 0,02°, Schrittzeit 2,4 Sekunden.

Probe: Glatte Oberfläche, eingefügt in einen Nickelprobenhalter. Die Probe wurde bei Raumtemperatur gemessen und aufbewahrt. In die Messungen wurden die Proben als leicht gepresste Pulverscheibe eingesetzt. Vor den Messungen wurden die Materialproben sehr sanft unter Verwendung eines Achatmörsers und einer Achatschale gemörsert.

Die qualitativen Charakteristika des Beugungsmusters, d. h. die Peak-Positionen und relativen Intensitäten der gemessenen Proben werden (im gesamten Text) in einer Tabelle (D-Werte und Intensität sowie °2Th.) angegeben.

Die Ergebnisse der Messungen der entsprechenden Proben sind in den Figuren 1 und 2 sowie in den oben stehenden Tabellen angegeben.

## Patentansprüche

1. Trinatrium-Zink-Diethylentriaminpentaessigsäure (Zn-DTPA) in kristalliner Form.

2. Trinatrium-Zink-Diethylentriaminpentaessigsäure nach Anspruch 1, wobei das Pulver-Röntgen-Diffraktogramm der Trinatrium-Zink-Diethylentriaminpentaessigsäure einem Pulver-Röntgen-Diffraktogramm zuzuordnen ist, das mindestens die folgenden charakteristischen Peaks aufweist (Form I):
| Pos. [°2Th.] | d-spacing [Å] | Rel. Int. [%] |
|---|---|---|
| 6.8836 | 14,90 | 48 |
| 8.1807 | 12,54 | 100 |
| 13.781 | 7,46 | 7 |
| 14.477 | 7,10 | 52 |
| 17.725 | 5,81 | 43 |
| 20.567 | 5,01 | 4 |
oder einem Pulver-Röntgen-Diffraktogramm zuzuordnen ist, das mindestens die folgenden charakteristischen Peaks aufweist (Form II):
| Pos. [°2Th.] | d-spacing [Å] | Rel. Int. [%] |
|---|---|---|
| | | |
| 11.302 | 9,08 | 21 |
| 11.384 | 9,02 | 86 |
| 14.892 | 6,90 | 32 |
| 16.699 | 6,16 | 100 |
| 21.721 | 4,75 | 41 |
| 33.734 | 3,08 | 10 |

3. Trinatrium-Zink-Diethylentriaminpentaessigsäure nach Anspruch 1 oder 2, wobei die Trinatrium-Zink-Diethylentriaminpentaessigsäure ausgehend von einem Wassergehalt von 8 Gew.-% bei Lagerung bei 65 % Luftfeuchtigkeit und 25 °C für 48 h ≤ 10 Gew.-% Wasser aufnimmt.

4. Trinatrium-Zink-Diethylentriaminpentaessigsäure nach einem der vorangehenden Ansprüche, wobei die Trinatrium-Zink-Diethylentriaminpentaessigsäure ausgehend von einem Wassergehalt von 8 Gew.-% bei Lagerung bei 65 % Luftfeuchtigkeit und 25 °C für 48 h ≤ 7 Gew.-% Wasser aufnimmt.

5. Trinatrium-Zink-Diethylentriaminpentaessigsäure nach einem der vorangehenden Ansprüche, wobei die Trinatrium-Zink-Diethylentriaminpentaessigsäure nach Lagerung bei 65 % Luftfeuchtigkeit und 25 °C für 200 h im festen Zustand verbleibt.

6. Trinatrium-Zink-Diethylentriaminpentaessigsäure nach einem der vorangehenden Ansprüche, wobei die Trinatrium-Zink-Diethylentriaminpentaessigsäure nach Lagerung bei 65 % Luftfeuchtigkeit und 25 °C für 200 h ≤ 16 Gew.-% Wasser umfasst.

7. Trinatrium-Zink-Diethylentriaminpentaessigsäure nach einem der vorangehenden Ansprüche, wobei die Trinatrium-Zink-Diethylentriaminpentaessigsäure nach Lagerung bei 65 % Luftfeuchtigkeit und 25 °C für 200 h ≤ 14 Gew.-% Wasser umfasst.

8. Trinatrium-Zink-Diethylentriaminpentaessigsäure nach einem der vorangehenden Ansprüche zur Verwendung als Arzneimittel.

9. Mensch und/oder Tier oral verabreichbare Formulierung, umfassend Trinatrium-Zink-Diethylentriaminpentaessigsäure nach einem der vorangehenden Ansprüche.

10. Trinatrium-Zink-Diethylentriaminpentaessigsäure nach einem der Ansprüche 1 bis 8 oder Mensch und/oder Tier oral verabreichbare Formulierung nach Anspruch 9 zur Behandlung oder Prophylaxe von Intoxikation mit Schwermetallen und/oder Radionucliden.

11. Verfahren zur Herstellung von Trinatrium-Zink-Diethylentriaminpentaessigsäure nach einem der Ansprüche 1 bis 8, umfassend die Schritte:
a) Reaktion von Tetraethylpentaessigsäure mit Zinkoxid oder einem anderen Zinksalz in Gegenwart von Wasser,
b) zweimaliges Einengen der Reaktionslösung auf ≤ 15 % des Ausgangsvolumens und nachfolgende Zugabe eines organischen Lösungsmittels und
c) Isolieren des kristallinen Reaktionsproduktes.

12. Verfahren zur Herstellung von Trinatrium-Zink-Diethylentriaminpentaessigsäure nach einem der Ansprüche 1 bis 8, umfassend die Schritte:
a) Reaktion von Tetraethylpentaessigsäure mit Zinkoxid oder einem anderen Zinksalz in Gegenwart von Wasser,
b) Entfernen von 90 bis 99 Gew.-% des Wassers aus dem Reaktionsgemisch, wobei 15 bis 100 Gew.-% organisches Lösungsmittel bezogen auf den Wassergehalt vor Beginn der Wasserentfernung parallel zur oder vor Beginn der Wasserentfernung zugesetzt wird,
c) ggf. Hinzufügen von weiterem organischem Lösungsmittel nach Schritt b),
d) weiteres Entfernen von Wasser bis die Dampftemperatur im Bereich von ± 5°C der Siedetemperatur des organischen Lösungsmittels liegt und/oder bis Kristallisation eintritt und
e) Isolieren des kristallinen Reaktionsproduktes.

13. Verfahren nach Anspruch 11 oder 12, wobei das organische Lösungsmittel ausgewählt ist, aus der Gruppe bestehend aus Aceton, n-Butanol, 2-Methyl-1-propanol, Methanol, Ethanol, n-Propanol, 2-Propanol, 2-Methyl-2-propanol, Toluol und Ethylacetat.

14. Verfahren nach Anspruch 11, wobei das organische Lösungsmittel Methanol ist oder Verfahren nach Anspruch 12, wobei das organische Lösungsmittel n-Butanol ist.

15. Verfahren nach einem der Ansprüche 11 bis 14, wobei das Einengen beziehungsweise die Entfernung von Wasser in b) durch azeotrope Destillation erfolgt.
